Europäisches Patentamt

European Patent Office

Office européen des brevets

(19)

(11) Publication number: **0 190 797**
**A2**

(12) # EUROPEAN PATENT APPLICATION

(21) Application number: 86200131.0

(22) Date of filing: 30.01.86

(51) Int. Cl.⁴: **A 01 N 33/12**
**A 61 K 31/44**

(30) Priority: 08.02.85 US 699891

(43) Date of publication of application:
13.08.86 Bulletin 86/33

(84) Designated Contracting States:
AT BE CH DE FR GB IT LI LU NL SE

(71) Applicant: Voit, John Kenneth
3 Airway Circle, Apartment 2A
Baltimore Maryland 21204(US)

(72) Inventor: Voit, John Kenneth
3 Airway Circle, Apartment 2A
Baltimore Maryland 21204(US)

(74) Representative: van der Beek, George Frans et al,
Nederlandsch Octrooibureau Johan de Wittlaan 15 P.O.
Box 29720
NL-2502 LS 's-Gravenhage(NL)

(54) Method for killing viruses and composition therefor.

(57) Viruses like Herpesvirus Type I and Type II are killed by contacting them with a viricidal composition comprising

| | |
|---|---|
| benzethonium chloride | 0.04 – 0.50 % by weight |
| ethanol | 15.0 – 25.0 % by weight |
| water | q. s. p. 100 % by weight |

and optionally a nonionic or cationic surfactant, sorbitol, glycerine, a flavorant or a colorant, for a period of time sufficient to kill the virus, typically less than 60 seconds. The composition is useful for rapidly disinfecting environmental surfaces.

EP 0 190 797 A2

TITLE:    METHOD FOR KILLING VIRUSES AND COMPOSITION THEREFOR

BACKGROUND OF THE INVENTION

Field of the Invention:

This invention relates to viricidal compostions and more particularly to aqueous solutions which kill viruses on environmental surfaces.

Description of the Prior Art:

Since the discovery that contagious infectious diseases are transmitted by the transfer of microorganisms from an infected person to a healthy one, efforts have been made to break the chain of contagion by destroying microorganisms in the environment. Simple scrubbing with soap or other detergent and water can markedly reduce the numbers of microorganisms on skin and environmental surfaces, and accordingly, thorough washing has long been a part of techniques for control of contagious diseases. However, washing is generally time consuming and, moreover, cannot be relied upon to kill all microorganisms. Consequently, other microbicidal compounds have been used, by themselves or in combination with washing, in antiseptic practice. Such materials as hexachlorophene, chlorhexidine, ortho-phenylphenol, cetylpyridinium chloride, and the like have come into common use for disinfecting skin and environmental surfaces.

While a number of anti-microbial compounds are known which are useful and generally effective against bacteria, the search for anti-viral compounds useful in disinfecting contaminated surfaces has not had the same success. Since viruses differ substantially from bacteria in structure and physiology, experience with anti-bacterial agents is not a certain guide in discovering and formulating agents which are capable of killing viruses on contaminated surfaces. Accordingly, an empirical approach has been adopted, and many conventional anti-microbial compounds have been tested in the hope that they will also show anti-viral activity.

In certain practical applications, the time required to kill a microorganism, and in particular a virus, is important. For example, if an environmental surface subject to contamination by contact with an infected person has to be immediately contacted by another person, rapid disinfection of the surface is necessary. A particular practical application of some importance arises in the current widespread education of many people in the techniques of cardiopulmonary resuscitation, and particularly in mouth-to-mouth resuscitation. In order to gain the practical experience needed, students practice the techniques using a mannequin which simulates a victim. Since several students use a single mannequin, some method of disinfecting the mouth area of the mannequin is needed to minimize the chance of transmitting disease. In particular, since herpesvirus has been found to be transmissible by contact with contaminated surfaces, it is particularly desirable to have an antiseptic agent which will assure rapid decontamination of environmental surfaces, e. g., in a contact time period of one minute or less.

Investigations of anti-viral disinfectants have hitherto focused on mere anti-viral activity, and have not devoted attention to the important problem of a rapid-killing antiviral. Even formulations developed specially to kill viruses, for example, a 7 % aqueous solution of phenol, have had to be left on the surfaces for some time in order to assure satisfactory killing of the viruses.

Various types of disinfectants have been tested for use as viricidal compounds, with varying degrees of success. McDuff, J. Assoc. Off. Anal. Chem. 59(5), pp. 1150-1156 (1976), discloses the use of quaternary ammonium compounds, phenolics, and iodophors as viricidal compounds in disinfecting of environmental surfaces.

Poli, G., et al., Arzneim. Forsch. 28(10), pp. 1172-1175 (1978) tested a number of quaternary ammonium compounds against six different types of virus, including herpesvirus. It was found that the viruses varied significantly in sensitivity to the different antivirals, some viruses being killed by certain of the compounds in one minute, others being resistant to a half-hour exposure. It is noteworthy that benzethonium chloride was not tested. Benzalkonium chloride, which bears some structural resemblance to benzethonium chloride, was found to be ineffective against herpesvirus in a concentration of 0.2 % when the contact time was five minutes, but effective at a contact time of thiry minutes.

Yagami, K., et al., Jikken Dobutsu (Japan) 31(1), pp. 27-35 (1982), tested the effectiveness of a number of compounds against feline herpesvirus and feline calicivirus. They found that benzethonium chloride is an effective agent against feline herpesvirus, capable of inactivating one strain of feline herpesevirus in a contact time of one minute when used as a disinfectant. This reference does not disclose use of benzethonium chloride for killing human herpesvirus Type I or Type II.

Sherrill, U. S. Patent 4,262,007, discloses treatment of herpesvirus infections by topical application of a composition comprising captan and benzethonium chloride. However, this discloure is concerned with treatment of viral lesions, not rapid decontamination of surfaces.

Martin, U. S. Patent 3,703,583, and Pensak et al., U. S. Patent 3,864,472, disclose mouthwash compositions containing benzethonium chloride and alcohol. However, these patents do not discuss viricidal activity of these compositions, and do not suggest that certain formulations of benzethonium chloride and ethanol might have rapid viricidal activity.

Therefore, a need has continued to exist for a viricidal composition which will rapidly and effectively kill viruses on enviromental surfaces.

## SUMMARY OF THE INVENTION

Accordingly, it is an object of this invention to provide a method for rapidly killing viruses on environmental surfaces.

A further object is to provide a method for assuring that environmental surfaces are free of contamination by viruses.

A further object is to provide a method of assuring that environmental surfaces are free of contamination by Herpes simplex, Type I or Type II.

A further object is to provide a viricidal composition.

A further object is to provide an aqueous composition which can rapidly kill viruses on environmental surfaces.

A further object is to provide a viricidal composition which can rapidly kill Herpes simplex, Type I and Type II.

Additional objects of the invention will become apparent from the description of the invention which follows.

The objects of the invention are achieved by a process for killing viruses comprising contacting the virus with a viricidal composition comprising benzalkonium chloride in an amount from about 0.4 % by weight to about 0.5 % by weight, ethanol in an amount from about 15 % by weight to about 25 % by weight, balance water. Herpes viruses, both type I and type II, on environmental surfaces are killed within 60 seconds by contact with this solution.

DETAILED DESCRIPTION OF THE INVENTION AND PREFERRED EMBODIMENTS

In the viricidal method of this invention the viricidal composition is applied to the virus-contaminated surface by conventional means such as swabbing or spraying, and allowed to stand for at least about 30 seconds, preferably 60 seconds or less. This contact time is suffucient to kill herpesvirus and the composition may then be removed. Since the ingredients are generally harmless to higher animals and humans the composition can be left on the surface and the volatile ingredients will evaporate.

The benzethonium chloride ingredient of the viricidal composition of this invention, benzethonium chloride, may be present in amounts of from about 0.04 % to about 0.5 % by weight. It is preferable that the composition contain at least about 0.05 % by weight of benzethonium chloride.

The amount of ethanol in the composition of this invention may range from 15 % to 25 % by weight. Preferably the composition contains about 18 % ethanol by weight.

Conventional ingredients can be added to the composition such as surfactants, flavorants, colorants, auxiliary ingredients the like.

The surfactant used should not react with the cationic quaternary ammonium compound, benzethonium chloride, which is an active ingredient of the composition. Accordingly, nonionic or cationic surfactants should be used. Preferred nonionic surfactants include polyoxyethylated derivatives of sorbitan mono- di- and trifatty acid esters wherein the fatty acid component has between 12 and 24 carbon atoms, and the polyethylene oxide chains contain from about 4 to about 30 ethylene oxide units and at least about 40 percent of the molecular weight of the molecule is ethylene oxide . Representative surfactants of this type include polyoxyethylene sorbitan monolaurate, polyoxyethylene sorbitan monopalmitate, polyoxyethylene sorbitan monostearate, polyoxyethylene sorbitan tristearate, polyoxyethylene sorbitan monooleate, polyoxyethylene sorbitan trioleate and the like. Cationic surfactants may include dimethylditallowammonium chloride, trimethylcetylammonium chloride, cetyldimethylbenzylammonium chloride, dimethyldicocoammonium chloride, condensation products of primary and secondary amines with ethylene oxide, and the like. The surfactant may be present in a proportion of from about 0.1 percent by weight to about 5 % by weight.

Additional auxiliary ingredients may be added to the viricidal composition of the invention to enhance the viricidal activity. Such compounds as glycerine and sorbitol may be added for this purpose. For example, glycerine may be added in amounts of from about 0.2 % to about 2 % by weight. Sorbitol may be added in amounts of of 0.2 to about 2 % by weight.

A sweetening agent, e. g., sodium saccharin may be added to the composition in an amount of from about 0.01 to about 0.1 percent by weight. Other conventional flavoring agents may also be added in amounts from about 0.05 to about 0.5 percent by weight. The purpose of these ingredients is to give the composition a pleasant flavor when used on surfaces which immediately come into contact with the mouth or lips. An example

The invention will be illustrated by the following examples which are not intended to be limiting. All percentages and parts

are by weight.

## EXAMPLE 1

This example illustrates the preparation of a preferred composition of the invention.

A viricidal solution was prepared having the following composition:

| | |
|---|---|
| Special denatured alcohol 38B | 18.00 % |
| Benzethonium chloride | 0.05 % |
| Flavor | 0.20 % |
| Sodium saccharin | 0.10 % |
| Sorbitol | 0.50 % |
| Polysorbate 20 | 2.00 % |
| Glycerine | 0.50 % |
| Water | 78.65 % |

The solution was prepared by first adding the water to the mixing tank, and then with agitation adding the saccharin, sorbitol and glycerine  The alcohol, flavor, polyoxyethylenesorbitan monolaurate and benzethonium chloride were mixed in a separate vessel and then added to the aqueous solution with agitation to prepare a clear solution.

## EXAMPLE 2

This example illustrates the viricidal effectiveness of the composition of this invention against Herpes simplex Type I.

A virus pool for testing was prepared by  growing Herpes simplex virus Type I in tissue culture (Hep-2 cells) by inoculating a cell culture with virus-containing fluid from a previous virus pool.  The cells were fed with a suitable medium and were observed by daily microscopic examination stationary at 37°C for cytopathic effects such as rounding, giant cells, pyknosis, granulation, syncytia and the like, for a period of up to ten days. To simulate organic soil for the tests, 5 % by weight of serum was added to the tissue culture.

In the test of viricidal efficacy, 0.2 ml of the virus pool was spread onto the flat glass bottom surface of each of a series of Petrie dishes, and allowed to dry to a film at 35°C for 30-45 minutes. The viricidal solution prepared in Example 1 was then sprayed over the virus film until the surface was wet with the test solution, and was allowed to remain in contact with the virus film for 30 seconds at 20-25°C. After 30 seconds, enough tripticase soy broth (TSB) was added to the dishes to achieve a total volume of solution of 2.0 ml. The mixture was removed by pipet and diluted in TSB up to $10^{-5}$ to $10^{-7}$. Decimal dilutions were then prepared and inoculated into a tissue culture, and the cultures were inspected at suitable intervals for the presence of virus.

Controls were run using surfaces untreated with the viricide, and toxicity controls were run wherein no virus was placed on the surface. The mean of four replications showed a decrease in viral activity of greater than $10^{-4.3}$, indicative of greater than 99.9 % inactivation of the virus

## EXAMPLE 3

This example illustrates the effectiveness of the viricidal composition of this invention against Herpes simplex Type II.

The test of Example 2 was repeated using Herpes simplex Type II in place of the Herpes simplex Type I of that example.

The results showed a greater than 99.9 % inactivation of the virus on an envirnmental surface by contact with the viricidal solution of this invention for less than 30 seconds.

The invention having now been fully described, it should be understood that it may be embodied in other specific forms or variations without departing from its spirit or essential characteristics. Accordingly, the embodiments described above are to be considered in all respects as illustrative and not restrictive; the scope of the invention being indicated by the appended claims rather than by the foregoing description, and all changes which come within the meaning and range of equivalency of the claims are intended to be embraced therein.

WHAT IS CLAIMED IS:

1.  A method for killing viruses like Herpes simplex virus comprising contacting said virus with a viricidal composition comprising

| | |
|---|---|
| benzethonium chloride | 0.04 - 0.10 % by weight |
| ethanol | 15.0 - 25.0 % by weight |
| water | q. s. p. 100 % by weight |

for a period of time sufficient to kill said virus.

2.  The method of Claim 1 wherein said period of time is no longer than one minute.

3.  The method of Claim 1 wherein said period of time is no longer than 30 seconds.

4.  The method of Claim 1 wherein said composition additionally comprises from about 1 % to about 4 % by weight of a surfactant selected from the group consisting of nonionic surfactants and cationic surfactants.

5.  The method of Claim 1 wherein said composition additionally comprises from about 0.3 % to about 1.0 % by weight of sorbitol.

6.  The method of Claim 1 wherein said composition additionally comprises from about 0.3 % to about 1.0 % by weight of glycerine.

0190797

7. A method of assuring that an environmental surface is free of contamination by viruses like Herpes simplex virus comprising contacting said surface for a period not greater than 60 seconds with a viricidal solution having the composition

| | |
|---|---|
| benzethonium chloride | 0.04 - 0.10 % by weight |
| ethanol | 15.0 - 25.0 % by weight |
| water | q. s. p. 100 % by |

8. The method of Claim 7 wherein said period of time is no longer than 30 seconds.

9. The method of Claim 7 wherein said composition additionally comprises from about 1 % to about 4 % by weight of a surfactant selected from the group consisting of nonionic surfactants and cationic surfactants.

10. The method of Claim 7 wherein said composition additionally comprises from about 0.3 % to about 1.0 % by weight of sorbitol.

11. The method of Claim 7 wherein said composition additionally comprises from about 0.3 % to about 1.0 % by weight of glycerine.

12. A viricidal composition comprising

| | |
|---|---|
| benzethonium chloride | 0.04 - 0.10 % by weight |
| ethanol | 15.0 - 25.0 % by weight |
| water | q. s. p. 100 % by weight. |

13. The viricidal composition of Claim 12 additionally comprising an auxiliary ingredient selected from the group consisting of a surfactant, a flavorant and a colorant.

14. The composition of Claim 12 wherein said composition additionally comprises from about 1 % to about 4 % by weight of a surfactant selected from the group consisting of nonionic surfactants and cationic surfactants.

15. The viricidal composition of Claim 12 wherein said composition additionally comprises from about 0.3 % to about 1.0 % by weight of sorbitol.

16. The viricidial composition of Claim 12 wherein said composition additionally comprises from about 0.3 % to about 1.0 % by weight of glycerine.

17.  The viricidal composition of Claim 12 having the composition:

| | |
|---|---|
| Special denatured alcohol 38B | 18.00 % |
| Benzethonium chloride | 0.05 % |
| Flavor | 0.20 % |
| Sodium saccharin | 0.10 % |
| Sorbitol | 0.50 % |
| Polysorbate 20 | 2.00 % |
| Glycerine | 0.50 % |
| Water | 78.65 % |